# EUROPEAN PATENT SPECIFICATION

(11) **EP 3 191 639 B1**
(45) Date of publication and mention of the grant of the patent: **27.04.2022**
(21) Application number: 15762952.8
(22) Date of filing: 31.08.2015
(51) Int. Cl.: D06F 58/10, A61L 2/00, A61L 2/10, A61L 9/00, D06F 58/20, D06F 58/38, D06F 103/32, D06F 105/28, D06F 105/30

(54) **A GARMENT CARE DEVICE FOR DRYING AND SANITIZING GARMENTS**
KLEIDERPFLEGEVORRICHTUNG ZUM TROCKNEN UND DESINFIZIEREN VON KLEIDERN
DISPOSITIF DE SOIN PERMETTANT DE SÉCHER ET D'ASSAINIR DES VÊTEMENTS

(30) Priority: 12.09.2014 EP 14184520
(43) Date of publication of application: 19.07.2017
(73) Proprietor: Koninklijke Philips N.V., 5656 AG Eindhoven (NL)
(72) Inventor: GAO, Peng, NL-5656 AE Eindhoven (NL); XIAO, Taikang, NL-5656 AE Eindhoven (NL)
(74) Representative: Kapoor, Pavan Puneet
(86) International application number: PCT/EP2015/069812
(87) International publication number: WO 2016/037873

(56) References cited:
- EP-A1- 0 627 519
- WO-A2-2008/013382
- WO-A2-2009/020305
- WO-A2-2009/020311
- DE-B- 1 128 601
- JP-A- 2001 029 698
- KR-B1- 101 321 527
- US-A- 3 475 828
- US-A- 3 513 669
- US-A- 4 625 432
- US-A- 5 152 077

## Description

### FIELD OF THE INVENTION

The invention relates to the field of garment care devices.

### BACKGROUND OF THE INVENTION

With the goal of maintaining a clean and hygienic domestic environment, various cleaning and sterilisation appliances are available. Such devices include sterilisers for grooming utensils, tooth brushes, cooking utensils and infant feeding equipment. However, devices for hygienic sterilisation of garments are less common.

Conventional washing machines do not typically reach the temperatures required to effectively sanitise clothing, meaning bacteria may survive the washing process and continue to grow on the garments. There are commercially available sterilising washing machines that provide cold water with ozone. However such devices suffer the draw back that any remaining ozone residue may cause an environmental safety hazard if it escapes into the local environment. Washing machines are also available that provide a wash cycle capable of heating water up to around 95 degrees Celsius. However, a conventional domestic water supply, which typically contains a number of impurities, is not ideal for cleaning clothes even at high temperature. Such devices typically also consume a large volume of water and are of a bulky size.

JP08338659 discloses a locker for sterilizing and drying clothes. The locker comprises a hanger on which a garment is hung, a heater to heat the garment, and a fan to circulate air within the locker. The air flows through into the top of a chamber, flows over the surface of the garment to dry the garment, and then flows out of the bottom of the chamber.

KR 101 321 527 B1 discloses a drier utilizing a heat exchanger to improve drying efficiency by properly utilizing waste heat.

WO 2008/013382 A2 discloses a dryer for drying e.g. clothes, having a heat pump that circulates relatively low temperature air to cabinet to dry laundry.

EP 0 627 519 A1 discloses a drier for clothing, footwear sporting and athletic equipment having air circulating between drying and mixing chambers, air is purged from and fresh air added into mixing chamber.

However, the structure of this known device does not allow a high drying and sanitizing effect of the garments. Moreover, it cannot dry nor sanitize garments which are folded.

### SUMMARY OF THE INVENTION

It is an object of the invention to provide a garment care device which substantially alleviates or overcomes the problems mentioned above.

The invention is defined by the independent claims. The dependent claims define advantageous embodiments.

According to the invention, there is provided a garment care device comprising: a housing; a chamber within the housing to receive at least one garment, the chamber having an air inlet and an air outlet; an air channel linking the air inlet and the air outlet; a first fan for circulating an air flow between the chamber, the air outlet, the air channel, and the air inlet; and a heater for heating the air flow, wherein the air inlet is configured such that air entering the chamber flows along a first direction, and wherein the air outlet (18) is configured to draw the air in the chamber along a second direction transverse to the first direction. The garment care device comprises a second fan and an extraction channel which vents to atmosphere and links the chamber with the second fan. The second fan is operable such as to selectively draw an air flow out of the chamber and into and through the extraction channel to vent to the atmosphere when the second fan is being operated. The garment care device further comprises a controller configured to control the heater and the first fan such that for a first predetermined time period the air in the garment care device is at above a first temperature level, and is circulated at above a first flow rate, and for a second predetermined time period the air is below the first temperature level and is circulated at above the first flow rate.

Therefore, the second fan can be switched on to remove water vapour from the chamber. The extraction channel vents to atmosphere such that when the second fan is switched on water vapour in the chamber is drawn through the extraction channel and vented to atmosphere.

The first direction may be substantially horizontal and the second direction may be substantially vertical. The air inlet and the air outlet may be spaced in the second direction.

The garment care device may comprise an ultraviolet lamp. The ultraviolet lamp is configured to shine ultraviolet rays into the chamber to sanitize the garments. Therefore, the time it takes for the garment care device to sanitize garments is reduced. The ultraviolet lamp may be a UV-C lamp.

In one embodiment, the garment care device comprises a first internal wall disposed between the air channel and the chamber, and the air inlet is formed through the first internal wall. This helps to save space and therefore make the garment care device more compact. In one embodiment, the garment care device comprises a second internal wall that is located proximate to an end of the first internal wall and the air outlet is configured to draw the air in the chamber along the second direction towards the second internal wall. The garment care device may comprise rear, side, top, bottom and/or door walls. The first internal wall may comprise one of the rear, side, top, bottom and/or door walls and the second internal wall may comprise another one of the rear, side, top, bottom and/or door walls. The first and second internal walls may be perpendicular.

In one embodiment, the air inlet comprises at least one first inlet vent and at least one second inlet vent configured such that air has to flow further through the air channel from the air outlet to reach the at least one first inlet vent than to reach the at least one second inlet vent, and wherein the at least one first inlet vent has a larger cross-sectional area than the at least one second inlet vent. Since the air must travel further through the air channel to reach the at least one first inlet vent than to reach the at least one second inlet vent, the flow resistance to the at least one first inlet vent will be higher and thus the flow rate therethrough will be lower. The larger cross-sectional area of the at least one first inlet vent compensates for this such that there is a more even distribution of the hot air entering the chamber. In one embodiment, the cross-sectional area of the at least one first inlet vent is increased by increasing the height thereof. The height may be defined as the in the second direction that the air flows through the chamber.

The garment care device may comprise a heater cover. The heater cover may be configured to diffuse the heat emitted by the heater such that the heat is more evenly distributed in the chamber to alleviate burning of the garments. The heater cover may be located between the heater and the chamber. In one embodiment, the heater cover is configured to form a heater space that links the chamber and the air channel, and the heater is received in the heater space. The heater may comprise one or more quartz tube infrared heat lamps or resistive heating elements.

The garment care device may comprise one or more reflective surfaces that are configured to reflect heat radiation in the chamber. The reflective surfaces may comprise a shiny metal, for example polished aluminium or stainless steel. The rear, side, top, bottom and/or door walls may comprise a reflective surface.

In one embodiment, the garment care device comprises at least one tray for receiving a garment. The garment may for example be folded flat and positioned on the at least one tray. The at least one tray may be removably mounted inside the chamber. The at least one tray may comprise an air impermeable structure. This advantageously enables the air in the chamber to flow through the or each tray and garments located thereon to reach the air outlet, rather than flowing around the at least one tray and any garments, and thus reduces the drying and sanitizing times of the garments and so increases the efficiency of the garment care device. In addition, the air permeable structure of the at least one tray saves space since it is not necessary to provide an air gap between the at least one tray and the walls of the chamber to enable air to pass through the chamber. The air permeable structure of the at least one tray may comprise at least one aperture through the thickness of the at least one tray or a mesh structure.

The garment care device may comprise a hanger that is configured such that a garment can be vertically hung in the chamber. The hanger may be removably mounted in the chamber.

The garment care device comprises a controller that is configured to control the heater and fan. The controller is configured to control the heater and fan such that for a first predetermined time period the air in the garment care device is at above a first temperature level and is circulated at above a first flow rate and for a second predetermined time period the air is below the first temperature level and is circulated at above the first flow rate. The advantage of this is that the residual heat of the air emitted during the first predetermined time period is used to dry the garments during the second predetermined time period, thereby increasing the efficiency of the garment care device. The heater may be switched off during the second predetermined time period or operated at a reduced power level.

In one embodiment, the garment care device comprises a temperature sensor that is coupled to the controller and is configured to measure the temperature of the air in the garment care device, and wherein the controller is configured to control the heater to keep the air circulating in the garment care device within a temperature range. In one such embodiment, the temperature range is between 50 to 120 degrees Celsius.

In one embodiment, the garment care device comprises a safety temperature sensor that is coupled to the controller and is configured to measure the temperature of the air in the garment care device, and wherein the controller is configured to switch off the heater and/or fan if the temperature measured by the safety temperature sensor exceeds a safety temperature level.

The garment care device may comprise a door. In one embodiment, the controller is configured to prevent the door from being opened if the heater, fan and/or ultraviolet lamp of the garment care device is powered. In an alternative embodiment, the controller is configured to switch off the heater, fan and/or ultraviolet lamp if the door is opened. This prevents the user from being burnt by the heat emitted from the heater and/or prevents the user from being exposed to potentially damaging ultraviolet rays emitted by the ultraviolet lamp.

These and other aspects of the invention will be apparent from and elucidated with reference to the embodiments described hereinafter.

### BRIEF DESCRIPTION OF THE DRAWINGS

Embodiments of the invention will now be described, by way of example only, with reference to the accompanying drawings, in which:
Fig. 1 is a perspective view of a garment care device according to an embodiment of the invention, having a door in a closed position;
Fig. 2 is a perspective view of the garment care device of Fig. 1, with the door in an open position;
Fig. 3 is a cross-sectional perspective view of the garment care device of Fig. 1;
Fig. 4 is a cross-sectional side view of the garment care device of Fig. 1;
Fig. 5 is a front view of a rear wall of the garment care device of Fig. 1;
Fig. 6 is a schematic side view of the airflow in the garment care device of Fig. 1;
Fig. 7 is a block diagram of a control system of the garment care device of Fig. 1;
Fig. 8 is a flow chart illustrating an exemplary drying program of the garment care device of Fig. 1; and,
Fig. 9 is a flow chart illustrating an exemplary sanitizing program of the garment care device of Fig. 1.

### DETAILED DESCRIPTION OF THE EMBODIMENTS

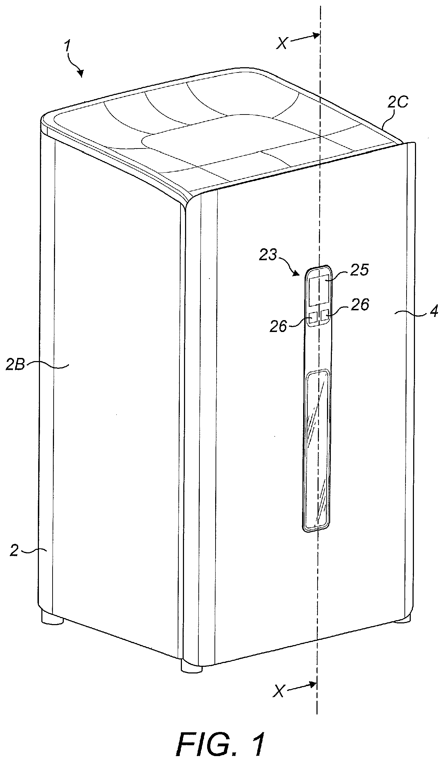

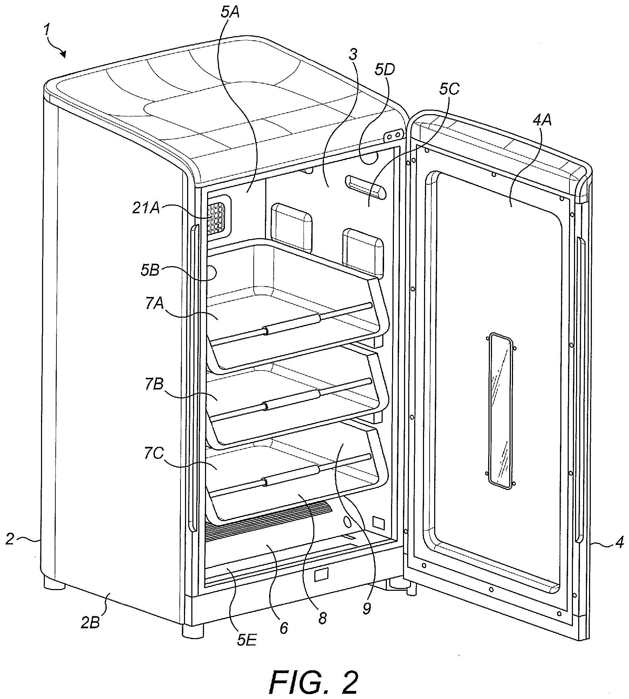

Referring to Figs. 1 to 9, a garment care device 1 of an embodiment of the invention is shown. The garment care device 1 comprises a housing 2 with an opening 3. The garment steamer 1 further comprises a door 4 that is connected to the housing 2 via a hinge and can be opened allow a user to access the interior of the housing 2 via the opening 3.

The housing 2 comprises an exterior rear wall 2A and opposing exterior side walls 2B, 2C and the door 4 comprises a door wall 4A. The exterior rear and side walls 2A, 2B, 2C and the door wall 4A are generally planar such that when the door 4 is closed the garment care device 1 is generally parallelepiped in shape (as shown in Fig. 1).

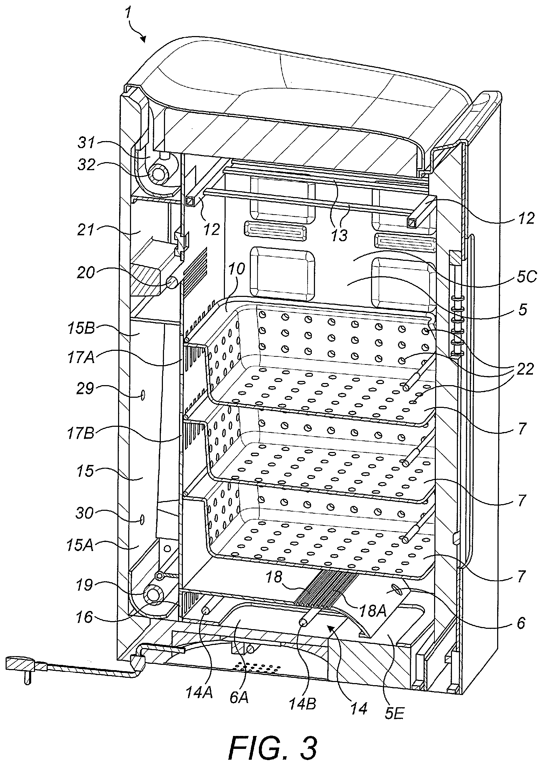

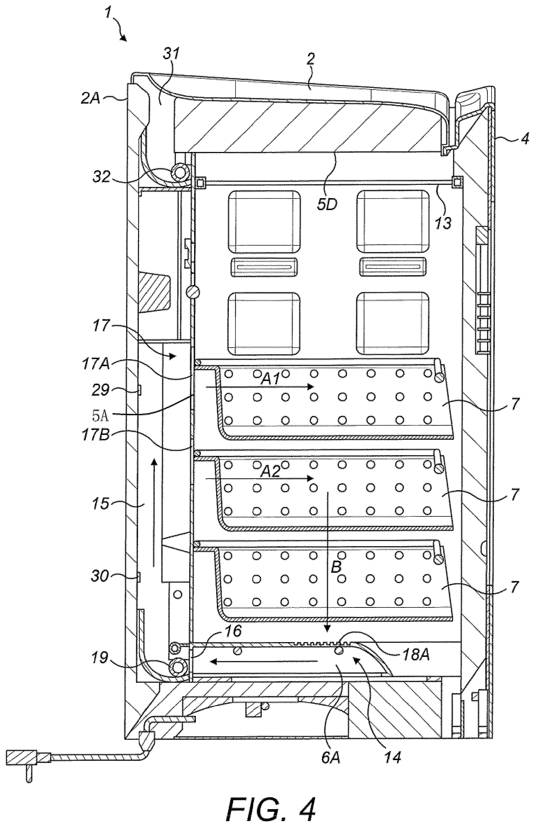

Figs. 3 and 4 show cross-sectional views of the garment care device 1 along the line X-X as shown in Fig. 1 and shows the garment care device 1 comprises a chamber 5 within the housing 2. The chamber 5 is generally rectangular prism shaped and is defined by an interior wall 5A (at the rear of chamber 5), opposing interior side walls 5B, 5C, an interior top wall 5D, an interior bottom wall 5E and a heater cover 6. The chamber 5 is sealed by the door wall 4A when the door 4 is closed.

A plurality of trays 7 are located in the chamber 5. In the present embodiment, the garment care device 1 comprises three trays 7 that are positioned sequentially in the chamber 5 to form upper, intermediate and lower trays 7A, 7B, 7C. Each of the trays 7 comprises a planar base 8 and a peripheral wall 9 that is upstanding from the planar base 8. The trays 7 are configured such that garments can be folded and placed on the planar base 8 of each tray 7 in the chamber 5. The peripheral wall 9 of each tray 7 comprises a lip 10 that is slidably received in corresponding grooves (not shown) in the interior side walls 5B, 5C of the chamber 5 such that the trays 7 can be removably mounted in the chamber 5.

A hanger 11 is provided towards the top of the chamber 5. The hanger 11 comprises a pair of support members 12 which are arranged in parallel and a plurality of longitudinal members 13 that extend between the support members 12 and are fixed thereto. The ends of the support members 12 are slidably received in corresponding grooves (not shown) in the interior side walls 5B, 5C of the chamber 5, proximate to the interior top wall 5D of the chamber 5, such that the hanger 11 can be removably mounted in the chamber 5. The hanger 11 is configured such that garments (not shown) can be vertically hung from the longitudinal members 13 in the chamber 5.

The heater cover 6 comprises a sheet of material, for example metal, that has a first end attached to the interior wall 5A. The sheet of material extends towards the door 4 and is bent downwardly such that a second end of the sheet of material is attached to the interior bottom wall 5E. Therefore, a heater space 6A is defined between the heater cover 6 and the interior bottom wall 5E.

A heater 14 is disposed in the heater space 6A. The heater 14 comprises first and second heating elements 14A, 14B. In the present embodiment, the first and second heating elements 14A, 14B are in the form of quartz tube infrared heat lamps 14A, 14B having power ratings of 200W and 400W respectively. However, it shall be recognized that other power ratings of the first and second heating elements 14A, 14B are intended to fall within the scope of the invention and that the number and/or type of heating elements may be varied.

The exterior rear wall 2A of the housing 2 is spaced from the interior wall 5A such that a passage 15 is formed therebetween. An opening 16 is provided in the interior wall 5A, proximate to the interior bottom wall 5E. The opening 16 fluidly communicates a first end 15A of the passage 15 with the heater space 6A. The chamber 5 comprises an inlet 17 that is located in the interior wall 5A and fluidly communicates a second end 15B of the passage 15 with the chamber 5.

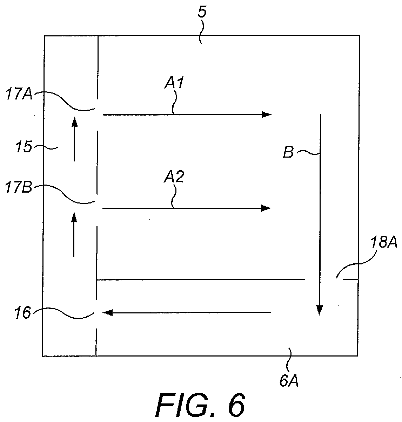

The inlet 17 comprises upper and lower inlet vents 17A, 17B. The upper inlet vents 17A are configured to direct air from the passage 15 onto garments on the upper tray 7A in the chamber 5 such that air flowing out of the upper inlet vents 17A travels across the chamber 5 to penetrate any garments (not shown) folded on the upper tray 7A. The direction of the air flowing out of the upper inlet vents 17A and into the chamber 5 is shown by arrow 'A1' in Figs. 4 and 6. The lower inlet vents 17B are configured to direct air from the passage 15 onto garments on the intermediate tray 7B such that air flowing out of the lower inlet vents 17B travels across the chamber 5 to penetrate any garments (not shown) folded on the intermediate tray 7B. The direction of the air flowing out of the lower inlet vents 17B and into the chamber 5 is shown by arrow 'A2' in Figs. 4 and 6.

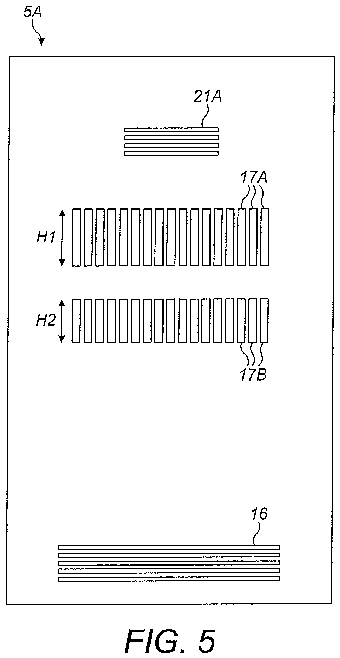

The upper inlet vents 17A have a larger cross-sectional area than the lower inlet vents 17B such that the flow resistance through the passage 15 to the upper inlet vents 17A is smaller than the flow resistance through the lower inlet vents 17B. This is to compensate for the increased length of the flow path that air must travel to flow through the upper inlet vents 17A and thus the increased flow resistance of said flow path and thus lower flow rate. Therefore, the flow rate of air flowing out of the upper and lower inlet vents 17A, 17B is substantially equal. In the present embodiment, the difference between the cross-sectional areas of the upper and lower inlet vents 17A, 17B is achieved by the height HI of the upper inlet vents 17A, in the direction between the interior top wall 5D and bottom wall 5E being larger than the height H2 of the lower inlet vents 17B (as shown in Fig. 5).

The chamber 5 comprises an outlet 18 that is located in the heater cover 6. The outlet 18 comprises a plurality of outlet vents 18A that fluidly communicate the chamber 5 with the heater space 6A.

A fan 19 is located in the passage 15 and is driven by a motor (not shown). The fan 19 is a cross-flow fan. However, other types of fan 19 are intended to fall within the scope of the invention. The fan 19 is configured to draw air from the chamber 5, via the outlet vents 18A, into the heater space 6A and then into the passage 15, via the opening 16 in the interior wall 5A. The air in the passage 15 is then conveyed by the fan 19 from the first end 15A of the passage 15 to the second end 15B. When the air reaches the second end 15B of the passage 15 it is expelled into the chamber 5 via the upper and lower inlet vents 17A, 17B in the direction shown by arrows 'A1' and 'A2' respectively such that the air penetrates into the garments (not shown) located on the upper and intermediate trays 7A, 7B. The air is then drawn towards the outlet vents 18A such that the air travels downwardly through the chamber 5 in the direction of arrow 'B' in Fig. 4. Therefore, the air is circulated by the fan 19 from the chamber 5, through the heater space 6A and passage 15 and back into the chamber 5. The garment care device 1 is therefore a closed circulation system.

Preferably, the garment care device 1 further comprises an ultraviolet lamp 20 is to radiate inside the chamber 5. For example, the UV lamp is located in a cavity 21 between the exterior and interior rear walls 2A, 5A. The cavity 21 is located above the passage 15. A window 21A is provided in the interior wall 5A such that the ultraviolet lamp 20 shines ultraviolet rays through the window 21A and into the chamber 5 to sterilize garments (not shown) located in the chamber 5. The ultraviolet lamp 20 maybe a UV-C lamp.

To dry garments (not shown), the user opens door 4 of the garment care device 1 and hangs the garments on the longitudinal members 13 of the hanger 11. The trays 7 can be removed from the chamber 5 such that there is more space in the chamber 5 to accommodate the garments. The garment care device 1 is then connected to a power supply (not shown) and switched on, and a drying program is selected.

During the drying program the heater 14 and the motor (not shown) of the fan 19 are powered. The fan 19 draws air into the heater space 6A and over the heater 14, which heats the air. More specifically, the first and second heating elements 14A, 14B are configured to emit infrared radiation to heat the air in the heater space 6A and to heat the heater cover 6. The heater cover 6 is configured to prevent the heater 14 from burning the garments in the chamber 5 by heating the garments directly. More specifically, the heater cover 6 is configured such that the majority of the infrared radiation emitted from the heater 14 cannot shine directly on the garments and instead shines onto the heater cover 6 or the interior bottom wall 5E. A proportion of the infrared radiation emitted by the heater 14 may travel through the outlet vents 18A in the heater cover 6 to heat the garments directly, however this proportion is not large enough to burn the garments. In an alternative embodiment, the heater cover 6 is configured such that substantially none of the infrared radiation emitted by the heater 14 directly reaches the garments in the chamber 5. This may be achieved be ensuring that there is no direct line-of-sight between the heater 14 and the garments in the chamber 5.

The radiation emitted by the first and second heating elements 14A, 14B that shines on the heater cover 6 heats the heater cover 6. The heat of the heater cover 6 is then transferred to the air in the heater space 6A and also is emitted as radiation in the chamber 5. A proportion of the radiation emitted by the heater cover 6 shines on the garments to heat the garments and a proportion of the radiation shines on the door wall 4A and the interior rear, side and top walls 5A, 5B, 5C, 5D wherein the radiation is reflected onto the garments. The heater cover 6 therefore acts to diffuse the radiation emitted by the first and second heating elements 14A, 14B such that the garments are evenly heated. To increase the amount of radiation that is reflected onto the garments the door wall 4A and the interior rear, side, top and walls 5A, 5B, 5C, 5D, 5E comprise a reflective material, for example a shiny material such as polished aluminium or stainless steel.

After the fan 19 has drawn air over the heater 14 to heat the air, the air is then drawn through the opening 16 in the interior rear wall 5A and into the passage 15 from the first end 15A of the passage 15 to the second end 15B. The heated airflow is then directed through the upper and lower inlet vents 17A, 17B and into the chamber 5 in the direction of arrows 'A1' and 'A2' respectively. The circulating hot air contacts the garment hung on the hanger 11 and therefore dries the garment. Since the air flowing out of the upper and lower inlet vents 17A, 17B travels transverse the chamber 5 the hot air penetrates the garments to reduce the drying time of the garments and thus increase the efficiency of the garment care device 1. The air that penetrates the garments then changes direction and is drawn downwardly through the garments in the direction of arrow 'B' towards the outlet vents 18A such that the drying time of the garments is further reduced. The circulating airflow helps to maintain an even temperature throughout the garments. When the drying program is finished, the heater 14 and fan 19 are switched off.

To sanitize garments (not shown), the user opens the door 4 of the garment care device 1 and the garments are folded and positioned on the planar base 8 of the trays 7. The hanger 11 can be removed from the chamber 5 such that there is more space in the chamber 5 to accommodate the garments. The garment care device 1 is then switched on and a sanitizing program is selected.

During the sanitizing program the heater 14, the motor of the fan 19 and the ultraviolet lamp 20 powered. The fan 19 draws air into the heater space 6A and over the heater 14 to heat the air in a similar manner to that described in respect of the drying program. The air is then drawn through the opening 16 in the interior rear wall 5A and into the passage 15, wherein the air flows along the passage 15 and into the chamber 5, via the upper and lower inlet vents 17A, 17B. The hot air permeates the garments and heats the garments to a temperature sufficient to kill any germs and bacteria that may be present, thereby sterilizing the garments. As described above, the upper and lower inlet vents 17A, 17B are configured such that the air enters the chamber 5 in a direction transverse the chamber 5 in the direction of arrows 'A1' and 'A2'. This causes the hot air to penetrate the garments located on the upper and intermediate trays 7A, 7B such that the temperature of the garments is increased and therefore the germs and bacteria are killed more quickly. In the present embodiment, the upper and lower inlet vents 17A, 17B are configured such that hot air entering the chamber 5 from the passage 15 flows substantially horizontally. However, it shall be recognised that the air may instead take a non-horizontal path, including a non-linear path, such that the air travels in a direction that is at an angle to the flow of air travelling through the chamber 5 towards the outlet 18 in the direction of arrow 'B'.

Each of the trays 7 comprise an air permeable structure, for example having a plurality of apertures 22 through the thickness of the planar base 8 and peripheral wall 9, such that hot air entering the garments disposed on the trays 7 can flow downwardly through the trays 7 and into the heating space 6A, via the outlet 18. For example, hot air that enters the chamber 5 from the upper inlet vent 17A penetrates the garments placed on the upper tray 7A and then travels downwardly through said garments and through the apertures 22 in the upper tray 7A. The hot air then penetrates the garments placed on the intermediate tray 7B and then travels downwardly through said garments and through the apertures 22 in the intermediate tray 7B. Subsequently, the hot air penetrates the garments placed on the lower tray 7C and then travels downwardly thorough said garments and through the apertures 22 in the lower tray 7C. Finally, the hot air flows out of the chamber 5 and into the heater space 6A, via the outlet vents 18A, wherein the air is reheated by the heater 14. Therefore, the hot air enters the chamber 5 from the inlet 17 in the direction of arrows 'A1' and 'A2' to permeate the garments on the upper and intermediate trays 7A, 7B and then changes direction to flow through the garments and trays 7 in the direction of arrow 'B' until the air reaches the outlet 18, wherein the air flows into the heater space 6A. In an alternate embodiment (not shown), the air permeable structure of the trays comprises a mesh.

At the same time that the air is heated in the heater space 6A and circulated through the garment care device 1 by the fan 19, the ultraviolet lamp 20 is powered to shine ultraviolet rays on the garments in the chamber 5. The ultraviolet rays kill germs and bacteria present on the garments to sterilize the garments. Therefore, the ultraviolet lamp 20 reduces the time it takes for the garment care device 1 to sterilize the garments.

Since the trays 7 are air permeable, it is not necessary to provide gaps between the trays 7 and the interior rear and side walls 5A, 5B, 5C and/or door wall 4A to allow for the air to flow from the inlet 17 to the outlet 18. This is advantageous since such gaps provide a flow path in the chamber 5 having a relatively low flow resistance and therefore hot air would tend to flow directly from the inlet 17 to outlet 18 via the gaps without flowing through the garments, which have a relatively high flow resistance, and so the hot air would not sufficiently penetrate the garments to sterilize the garments. In addition, since the hot air flows through the apertures 22 in the trays 7 in the direction of arrow 'B', rather than flowing along the surface of the trays 7 which would be the case if the trays 7 were air impermeable, then the contact between the trays 7 and the flow of hot air is reduced such that less heat is transferred from the air to the trays 7. Therefore, the efficiency of the garment care device 1 is increased. When the sterilizing program is finished, the heater 14, the motor of the fan 19 and the ultraviolet lamp 20 are switched off.

The garment care device 1 comprises a control unit 23 comprising a controller 24, a display 25 and one or more control buttons 26. The control buttons 26 allow for a user to manipulate a user interface displayed by the display 26 to input signals into the controller 24 to control the operation of the garment care device 1.

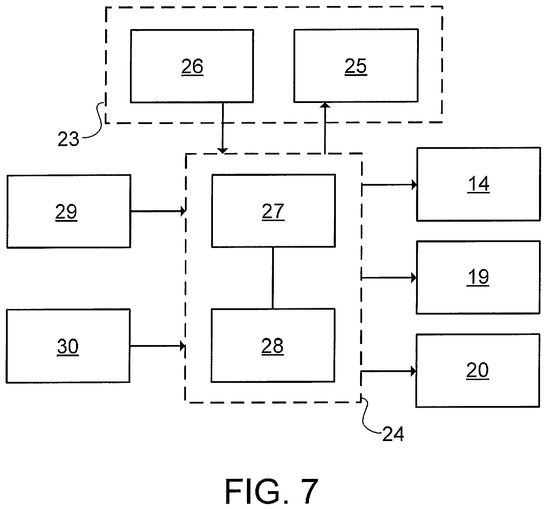

The controller 24 comprises a processor 27 and a memory 28 (as shown schematically in the block diagram of Fig. 7). The memory 28 includes one or more pre-set programs for operation of the garment care device 1.

The pre-set operation programs stored in the memory 28 of the controller 24 include the drying and sanitizing programs described above. The memory 28 may include single-garment and multi-garment drying and sanitizing programs, and different drying and sanitizing programs for different garment materials. Each program may have different parameters, for example, the single-garment program drying and sanitizing programs maybe shorter than the multi-garment drying and sanitizing programs, and a program for cotton garments may run at a higher temperature than a program for wool garments or other 'delicate' garments.

The controller 24 is also connected to the heater 14, the motor that drives the fan 19 and the ultraviolet lamp 20 to control the operation thereof. The garment care device 1 also includes a first temperature sensor 29 located within the passage 15. The first temperature sensor 29 is connected to the controller 24 so that the controller 24 can receive a temperature signal from the first temperature sensor 29 representing the detected temperature within the passage 15.

A user may operate the garment care device 1 using the control unit 23 to select a desired drying or sanitizing program. The controller 24 then controls operation of the heater 14, the motor of the fan 19 and ultraviolet lamp 20 in dependence on the program selected. During operation, the first temperature sensor 29 sends temperature signals to the controller 24. If the detected temperature within the passage 15 exceeds the desired operating temperature of the selected program, or is outside a permitted tolerance of the desired operating temperature, the controller 24 may turn off the heater 14, or reduce the power supplied to the heater 14, until the detected temperature falls to the desired operating temperature or within a permitted range of tolerance of the desired temperature. Also, the controller 24 may include a timer so that it can operate the garment care device 1 for a predetermined period of time in dependence on the selected operating program, and to stop operation of the garment care device 1 when the program concludes after the predetermined operating time has elapsed.

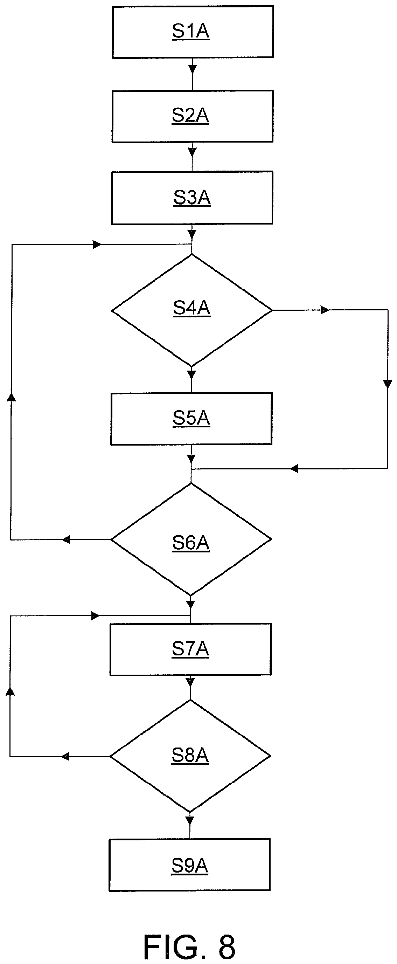

An exemplary operation of a drying program of the garment care device 1 of the present invention is shown schematically in the flow chart of Fig. 8. At step S1A, the drying program is started via a user selecting the desired program on the user interface of the control unit (not shown). At step S2A, the controller 24 sets the timer count to zero and begins the process timer. At step S3A, the motor is powered to operate the fan 19. At step S4A, the controller 24 uses the temperature signal from the first temperature sensor 29 to query whether the temperature within the passage 15 is greater than a predetermined maximum threshold temperature for the selected drying program. If the temperature is not above the maximum threshold temperature, the heater 14 is powered at step S5A and the process proceeds to step S6A. If the temperature is above the maximum threshold temperature, power to the heater 14 is stopped and the program proceeds to step S6A. At step S6A, the controller 24 queries whether the elapsed time since the start of the program has reached a first pre-set cycle duration time. If the first pre-set cycle duration time has not been reached the program loops back to step S4A. If the first pre-set cycle duration time has elapsed, the drying program moves to step S7A. At step S7A, the heater 14 is switched off and the fan 19 is operated such that the residual heat of the air is used to dry the garments, thereby increasing the efficiency of the garment care device 1, and the program proceeds to step S8A. At step S8A, the controller 24 queries whether the elapsed time since the start of the program has reached a second pre-set cycle duration time. If the second pre-set cycle duration time has not been reached the program loops back to step S7A. If the second pre-set cycle duration time has elapsed, the drying program is complete and so the process proceeds to step S9A which is the end of the drying program.

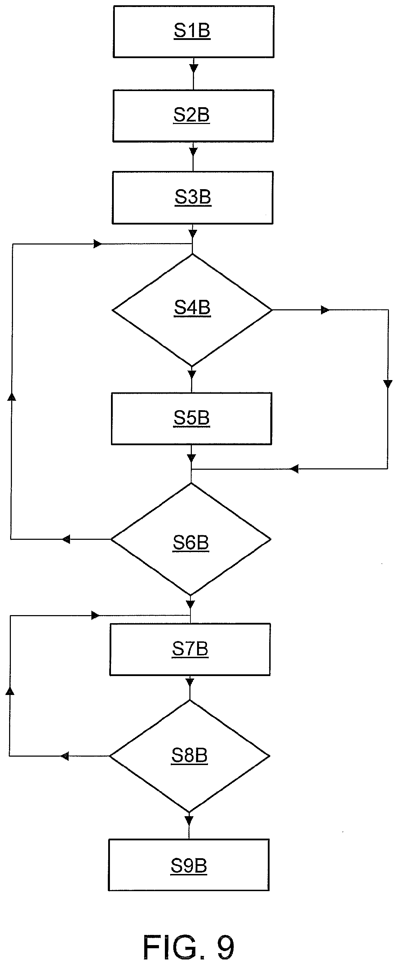

An exemplary operation of a sanitizing program of the garment care device 1 of the present invention is shown schematically in the flow chart of Fig. 9. At step S1B, the sanitizing program is started via a user selecting the desired program on the user interface of the control unit (not shown). At step S2B, the controller 24 sets the timer count to zero and begins the process timer. At step S3B, the motor is powered to operate the fan 19 and the ultraviolet lamp 20 is powered. At step S4B, the controller 24 uses the temperature signal from the first temperature sensor 29 to query whether the temperature within the passage 15 is greater than a predetermined maximum threshold temperature for the selected sanitizing program. If the temperature is not above the maximum threshold temperature, the heater 14 is powered at step S5B and the process proceeds to step S6B. If the temperature is above the maximum threshold temperature, power to the heater 14 is stopped and the program proceeds to step S6B. At step S6B, the controller 24 queries whether the elapsed time since the start of the program has reached a first pre-set cycle duration time. If the first pre-set cycle duration time has not been reached the program loops back to step S4B. If the first pre-set cycle duration time has elapsed, the drying program moves to step S7B. At step S7B, the heater 14 is switched off and the ultraviolet lamp 20 is powered and the fan 19 is operated such that the residual heat of the air is used to sanitizing the garments, thereby increasing the efficiency of the garment care device 1, and the program proceeds to step S8B. At step S8B, the controller 24 queries whether the elapsed time since the start of the program has reached a second pre-set cycle duration time. If the second pre-set cycle duration time has not been reached the program loops back to step S7B. If the second pre-set cycle duration time has elapsed, the drying program is complete and so the process proceeds to step S9B which is the end of the drying program.

The controller 24 may execute a safety shut-off function for the garment care device 1, by monitoring the temperature within the passage 15 during operation of the garment care device 1 using a second temperature sensor 30 that is disposed within the passage 15, and automatically stopping operation of the garment care device 1 if the detected temperature exceeds a maximum threshold safety temperature during the drying or sanitizing programs. Alternatively, the controller 24 may shut off power to the heater 14 but maintain operation of the fan 19 and/or ultraviolet lamp 20 until the detected temperature falls to a predetermined level, or within a predetermined temperature range. The second temperature sensor 30 provides a failsafe in the event that the first temperature 29 malfunctions and therefore prevents the garment care device 1 from overheating in the event of such a malfunction. In one embodiment, the first temperature sensor 29 is proximate to the second end 15B of the passage 15 and the second temperature sensor 30 is proximate to the first end 15A of the passage 15. In an alternative embodiment, the first and/or second temperature sensor 29, 30 is located in the chamber 5 or the heater space 6A.

The controller 24 may be configured to prevent the door 4 from being opened if the heater 14, fan 19 and/or ultraviolet lamp 20 are powered. In one such embodiment, the controller 24 is connected to a door lock (not shown), for example an electromechanical latch, that is configured to lock the door 4 when a locking signal is sent by the controller 24. Alternatively, the controller 24 may be configured to switch off the heater 14, fan 19 and/or ultraviolet lamp 20 if the door 4 is opened. In one such embodiment, the controller 24 is coupled to a sensor (not shown), for example a contact sensor, that detects when the door 4 is open. This prevents the user from being burnt by the heat emitted from the heater 14 and/or prevents the user from being exposed to potentially damaging ultraviolet rays emitted by the ultraviolet lamp 20.

The garment care device 1 comprises an extraction channel 31 that fluidly communicates the chamber 5 with the atmosphere outside the garment care device 1. The garment care device 1 further comprises a second fan 32 that is coupled to the controller 24. The controller 24 is configured to operate the second fan 32 to selectively draw fluid out of the chamber 5, and through the extraction channel 31, to vent to atmosphere. As the garments are dried by the hot air in the chamber, liquid water in the garments evaporates and accumulates as water vapour in the chamber 5. As the amount of water vapour in the air in the chamber 5 increases, the garment care device 1 becomes less efficient at drying the garments. To alleviate this, the second fan 32 can be operated by the controller 24 to draw air and water vapour out of the chamber 5 to vent to atmosphere. This will cause the pressure to drop in the chamber 5 and so fresh air will be drawn through an gap or aperture (not shown) that fluidly communicates the inside of the garment care device 1 with atmosphere. This process of venting the water vapour to atmosphere can be performed a predetermined period of time after the drying or sanitizing program has started. Alternatively, the garment care device 1 may comprise a water sensor (not shown), for example a capacitive or resistive water sensor, which is coupled to the controller 24 and measures the water vapour content in the chamber 5. If the water vapour content measured by the water sensor exceeds a predetermined level then the controller 24 operates the second fan 32 to vent some of the water vapour to atmosphere.

Although in the above described embodiment the passage 15 is disposed between the exterior rear wall 2A and the interior rear wall 5A, it should be recognised that the passage 15 may be positioned elsewhere in the garment care device 1. For example, the passage 15 may alternatively be disposed between one of the exterior side walls 2B, 2C and a corresponding interior side wall 5B, 5C. Alternatively, the passage 15 may be disposed in the door 4 of the garment care device 1.

Although in the above described embodiment the heater 14 is disposed in the heater space 6A, in an alternative embodiments (not shown) the heater cover 6 may be omitted such that the heater 14 is disposed in the chamber 5 or the heater 14 may be disposed in the passage 15. In one embodiment, the heater space 6A is disposed between the passage 15 and the inlet 17.

Although in the above described embodiment the heater 14 comprises first and second heating elements 14A, 14B each comprising a quartz tube infrared heat lamp, it should be recognised that the number and type of heating elements maybe varied. For example, in one alternative embodiment (not shown) the heater comprises a resistive heating element. The resistive heating element (not shown) may comprise a resistive heating wire that is arranged in a serpentine configuration and is bonded to an aluminium sheet. An electrical current is passed through the resistive heating wire to generate heat. The aluminium sheet may be bonded to the interior bottom wall 5E of the garment care device 1.

In the above described embodiment the garment care device 1 comprises upper, intermediate and lower trays 7A, 7B, 7C. However, it shall be recognised that the number of trays 7 may be varied. In one embodiment, the trays 7 and/or the hanger 11 are omitted.

In the above described embodiment the garment care device 1 comprises an air inlet 17 comprising upper and lower vents 17A, 17B. However, it will be recognised that in an alternative embodiment (not shown) one of the upper and lower vents 17A, 17B may be omitted. In yet another embodiment (not shown), the inlet further comprises vents that are disposed in the rear wall 5A and are configured to direct air onto garments placed on the lower tray 7C.

The above embodiments as described are only illustrative, and not intended to limit the technique approaches of the present invention. Although the present invention is described in details referring to the preferable embodiments, those skilled in the art will understand that the technique approaches of the present invention can be modified or equally displaced without departing from scope of the present invention, which will also fall into the protective scope of the claims of the present invention. In the claims, the word "comprising" does not exclude other elements or steps, and the indefinite article "a" or "an" does not exclude a plurality. Any reference signs in the claims should not be construed as limiting the scope.

## Claims

1. A garment care device (1) comprising:
- a housing (2);
- a chamber (5) within the housing (2) to receive at least one garment, the chamber (5) having an air inlet (17) and an air outlet (18);
- an air channel (15) linking the air inlet (17) and the air outlet (18);
- a first fan (19) for circulating an air flow between the chamber (5), the air outlet (18), the air channel (15), and the air inlet (17),
- a heater (14) for heating the air flow,
wherein the air inlet (17) is configured such that air entering the chamber (5) flows along a first direction (A1, A2), and wherein the air outlet (18) is configured to draw the air in the chamber (5) along a second direction (B) transverse to the first direction (A1, A2),
**characterised in that** the garment care device (1) comprises further
- an extraction channel (31) which vents to atmosphere,
- a second fan (32), the extraction channel (31) linking the chamber (5) with the second fan (32), wherein the second fan (32) is operable such as to selectively draw a further air flow out of the chamber (5), into and through the extraction channel (31) to vent to the atmosphere when the second fan is being operated, and
- a controller (24) configured to control the heater (14) and the first fan (19) such that for a first predetermined time period the air in the garment care device (1) is at above a first temperature level, and is circulated at above a first flow rate, and for a second predetermined time period the air is below the first temperature level and is circulated at above the first flow rate.

2. A garment care device (1) according to claim 1, comprising an ultraviolet lamp (20) to radiate inside the chamber (5).

3. A garment care device (1) according to claim 1 or 2, comprising a first internal wall (5A) disposed between the air channel (15) and the chamber (5), and wherein the air inlet (17) is formed through the first internal wall (5A).

4. A garment care device (1) according to claim 3, comprising a second internal wall (5E) that is located proximate to an end of the first internal wall (5A) and wherein the air outlet (18) is configured to draw the air in the chamber (5) along the second direction (B) towards the second internal wall (5E).

5. A garment care device (1) according to any one of the preceding claims, wherein the air inlet (17) comprises at least one first inlet vent (17A) and at least one second inlet vent (17B), wherein the at least one first inlet vent (17A) has a larger cross-sectional area than the at least one second inlet vent (17B).

6. A garment care device (1) according to any one of the preceding claims, comprising a heater cover (6) placed above the heater (14).

7. A garment care device (1) according to claim 6, wherein the heater cover (6) is configured to form a heater space (6A) linking the chamber (5) and the air channel (15), and wherein the heater (14) is received in the heater space (6A).

8. A garment care device (1) according to any one of the preceding claims, comprising a reflective surface (4A, 5A, 5B, 5C, 5D, 5E) in the chamber (5) for reflecting heat radiation in the chamber (5).

9. A garment care device (1) according to any one of the preceding claims, comprising at least one tray (7) for receiving said at least one garment.

10. A garment care device (1) according to claim 9, wherein the at least one tray (7) comprises an air permeable structure.

11. A garment care device (1) according to any one of the preceding claims, comprising a hanger (11) configured to vertically hang said at least one garment in the chamber (5).

12. A garment care device (1) according to claim 1, comprising a temperature sensor (29) coupled to the controller (24) and configured to measure the temperature of the air in the garment care device (1), and wherein the controller (24) is configured to control the heater (14) to keep the air circulating in the garment care device (1) within a given temperature range.

## Patentansprüche

1. Ein Bekleidungspflegegerät (1), das Folgendes umfasst:
- ein Gehäuse (2);
- eine Kammer (5) innerhalb des Gehäuses (2) zum Aufnehmen mindestens eines Kleidungsstücks, wobei die Kammer (5) einen Lufteinlass (17) und einen Luftauslass (18) umfasst;
- einen Luftkanal (15), der den Lufteinlass (17) und den Luftauslass (18) miteinander verbindet;
- ein erstes Gebläse (19) zum Zirkulieren des Luftstroms zwischen der Kammer (5), dem Luftauslass (18), dem Luftkanal (15) und dem Lufteinlass (17),
- ein Heizelement (14) zum Erwärmen des Luftstroms, wobei der Lufteinlass (17) so konfiguriert ist, dass die in die Kammer (5) eintretende Luft entlang einer ersten Richtung (A1, A2) strömt, und wobei der Luftauslass (18) so konfiguriert ist, dass die Luft in der Kammer (5) entlang einer zweiten Richtung (B) quer zur ersten Richtung (A1, A2) abgeführt wird, **dadurch gekennzeichnet, dass** das Bekleidungspflegegerät (1) zudem Folgendes umfasst:
- einen Abzugskanal (31), der in die Atmosphäre entlüftet,
- ein zweites Gebläse (32), wobei der Abzugskanal (31) die Kammer (5) mit dem zweiten Gebläse (32) verbindet, wobei das zweite Gebläse (32) so betrieben werden kann, dass es selektiv einen weiteren Luftstrom aus der Kammer (5) in und durch den Abzugskanal (31) leitet, um in die Atmosphäre zu entlüften, wenn das zweite Gebläse in Betrieb ist, und
- eine Steuerung (24), die das Heizelement (14) und das erste Gebläse (19) so steuert, dass über einen ersten vorab festgelegten Zeitraum die Luft im Bekleidungspflegegerät (1) oberhalb eines ersten Temperaturniveaus liegt und mit einer ersten Durchflussrate umgewälzt wird, während die Luft für einen zweiten vorab festgelegten Zeitraum unterhalb des ersten Temperaturniveaus liegt und mit einer höheren Durchflussrate umgewälzt wird.

2. Ein Bekleidungspflegegerät (1) gemäß Anspruch 1, das eine Ultraviolett-Lampe (20) umfasst, die das Innere der Kammer (5) bestrahlt.

3. Ein Bekleidungspflegegerät (1) gemäß Anspruch 1 oder 2, das eine erste Innenwand (5A) zwischen dem Luftkanal (15) und der Kammer (5) umfasst, und wobei sich der Lufteinlass (17) durch die erste Innenwand (5A) erstreckt.

4. Ein Bekleidungspflegegerät (1) gemäß Anspruch 3, das eine zweite Innenwand (5E) umfasst, die sich in der Nähe eines Endes der ersten Innenwand (5A) befindet, und wobei der Luftauslass (18) so konfiguriert ist, dass die Luft in der Kammer (5) entlang einer zweiten Richtung (B) in Richtung der zweiten Innenwand (5E) abgeführt wird.

5. Ein Bekleidungspflegegerät (1) gemäß einem der vorherigen Ansprüche, wobei der Lufteinlass (17) mindestens eine erste Einlassöffnung (17A) und mindestens eine zweite Einlassöffnung (17B) umfasst, wobei die mindestens eine erste Einlassöffnung (17A) eine größere Querschnittsfläche aufweist als die mindestens eine zweite Einlassöffnung (17B).

6. Ein Bekleidungspflegegerät (1) gemäß einem der vorherigen Ansprüche, das eine Heizelementabdeckung (6) umfasst, die über dem Heizelement (14) angeordnet ist.

7. Ein Bekleidungspflegegerät (1) gemäß Anspruch 6, wobei die Heizelementabdeckung (6) einen Heizraum (6A) bildet, der die Kammer (5) und den Luftkanal (15) verbindet, und wobei das Heizelement (14) im Heizraum (6A) aufgenommen wird.

8. Ein Bekleidungspflegegerät (1) gemäß einem der vorherigen Ansprüche, das in der Kammer (5) eine reflektierende Oberfläche (4A, 5A, 5B, 5C, 5D, 5E) zum Reflektieren der Wärmestrahlung in der Kammer (5) umfasst.

9. Ein Bekleidungspflegegerät (1) gemäß einem der vorherigen Ansprüche, das mindestens einen Einsatz (7) zum Aufnehmen des mindestens einen Kleidungsstücks umfasst.

10. Ein Bekleidungspflegegerät (1) gemäß Anspruch 9, wobei der mindestens eine Einsatz (7) eine luftdurchlässige Struktur aufweist.

11. Ein Bekleidungspflegegerät (1) gemäß einem der vorherigen Ansprüche, das einen Bügel (11) zum vertikalen Aufhängen des mindestens einen Kleidungsstücks in der Kammer (5) umfasst.

12. Ein Bekleidungspflegegerät (1) gemäß Anspruch 1, das einen Temperatursensor (29) umfasst, der mit der Steuerung (24) verbunden ist und die Temperatur der Luft im Bekleidungspflegegerät (1) misst, und wobei die Steuerung (24) das Heizelement (14) steuert, um die im Bekleidungspflegegerät (1) zirkulierende Luft innerhalb eines vorab festgelegten Temperaturbereichs zu halten.

## Revendications

1. Dispositif d'entretien des vêtements (1) comprenant :
- un logement (2) ;
- une chambre (5) à l'intérieur du logement (2) permettant de recevoir au moins un vêtement, la chambre (5) comportant une entrée d'air (17) et une sortie d'air (18) ;
- un canal d'air (15) reliant l'entrée d'air (17) et la sortie d'air (18) ;
- un premier ventilateur (19) destiné à la circulation d'un écoulement d'air entre la chambre (5), la sortie d'air (18), le canal d'air (15) et l'entrée d'air (17) ;
- un dispositif chauffant (14) destiné au chauffage de l'écoulement d'air,
dans lequel l'entrée d'air (17) est conçue de sorte telle que l'air entrant dans la chambre (5) s'écoule le long d'une première direction (A1, A2), et dans lequel la sortie d'air (18) est conçue pour aspirer l'air dans la chambre (5) le long d'une seconde direction (B) transversale à la première direction (A1, A2),
**caractérisé en ce que** le dispositif d'entretien des vêtements (1) comprend en outre
- un canal d'extraction (31), lequel renvoie l'air dans l'atmosphère,
- un second ventilateur (32), dans lequel le canal d'extraction (31) relie la chambre (5) au second ventilateur (32), dans lequel le second ventilateur (32) peut fonctionner de manière à aspirer sélectivement un écoulement d'air supplémentaire hors de la chambre (5), dans et à travers le canal d'extraction (31) pour renvoyer l'air vers l'atmosphère lorsque le second ventilateur fonctionne, et
- un dispositif de commande (24) conçu pour commander le dispositif chauffant (14) et le premier ventilateur (19) de telle sorte que pendant une première période de temps prédéterminée, la température de l'air dans le dispositif d'entretien des vêtements (1) est supérieure à un premier niveau de température, et l'air est mis en circulation lorsque son débit est inférieur à un premier débit, et pendant une seconde période de temps prédéterminée, la température de l'air est inférieure au premier niveau de température et l'air est mis en circulation lorsque son débit est supérieur au premier débit.

2. Dispositif d'entretien des vêtements (1) selon la revendication 1, comprenant une lampe ultraviolette (20) permettant de rayonner à l'intérieur de la chambre (5).

3. Dispositif d'entretien des vêtements (1) selon la revendication 1 ou 2, comprenant une première paroi interne (5A) disposée entre le canal d'air (15) et la chambre (5), et dans lequel l'entrée d'air (17) est formée à travers la première paroi interne (5A).

4. Dispositif d'entretien des vêtements (1) selon la revendication 3, comprenant une seconde paroi interne (5E), laquelle est située à proximité d'une extrémité de la première paroi interne (5A) et dans lequel la sortie d'air (18) est conçue pour aspirer l'air dans la chambre (5) le long de la seconde direction (B) vers la seconde paroi interne (5E).

5. Dispositif d'entretien de vêtements (1) selon l'une quelconque des revendications précédentes, dans lequel l'entrée d'air (17) comprend au moins un premier orifice d'entrée (17A) et au moins un second orifice d'entrée (17B), dans lequel l'au moins un premier orifice d'entrée (17A) présente une surface de section transversale supérieure à l'au moins un second orifice d'entrée (17B).

6. Dispositif d'entretien des vêtements (1) selon l'une quelconque des revendications précédentes, comprenant un couvercle (6) du dispositif chauffant placé au-dessus du dispositif chauffant (14).

7. Dispositif d'entretien des vêtements (1) selon la revendication 6, dans lequel le couvercle (6) du dispositif chauffant est conçu pour former un espace (6A) du dispositif chauffant reliant la chambre (5) et le canal d'air (15), et dans lequel le dispositif chauffant (14) est reçu dans l'espace (6A) du dispositif chauffant.

8. Dispositif d'entretien des vêtements (1) selon l'une quelconque des revendications précédentes, comprenant une surface réfléchissante (4A, 5A, 5B, 5C, 5D, 5E) dans la chambre (5) destinée à la réflexion du rayonnement thermique dans la chambre (5).

9. Dispositif d'entretien des vêtements (1) selon l'une quelconque des revendications précédentes, comprenant au moins un plateau (7) destiné à la réception dudit au moins un vêtement.

10. Dispositif d'entretien des vêtements (1) selon la revendication 9, dans lequel l'au moins un plateau (7) comprend une structure perméable à l'air.

11. Dispositif d'entretien des vêtements (1) selon l'une quelconque des revendications précédentes, comprenant un cintre (11) conçu pour suspendre verticalement ledit au moins un vêtement dans la chambre (5) .

12. Dispositif d'entretien des vêtements (1) selon la revendication 1, comprenant un capteur de température (29) couplé au dispositif de commande (24) et conçu pour mesurer la température de l'air dans le dispositif d'entretien des vêtements (1), et dans lequel le dispositif de commande (24) est conçu pour commander le dispositif chauffant (14) pour maintenir l'air circulant dans le dispositif d'entretien des vêtements (1) dans une plage de température donnée.
